# NOUVEAU FASCICULE DE BREVET EUROPEEN

(11) **EP 0 742 195 B2**
(45) Date de publication et mention de la décision concernant l'opposition: **08.03.2006**
(45) Mention de la délivrance du brevet: 20.09.2000
(21) Numéro de dépôt: 96400921.1
(22) Date de dépôt: 29.04.1996
(51) Int. Cl.: C07C 43/04, C07C 11/06, C07C 41/06, C07C 6/04

(54) **Procédé et installation pour la conversion de coupes C4 et C5 oléfiniques en éther et en propylène**
Verfahren und Anlage zur Umsetzung von C4 und C5 olefinischen Fraktionen in Ether und Propylenen
Process and installation for the conversion of C4 and C5 olefinic fractions into ethers and propylene

(30) Priorité: 11.05.1995 FR 9505561
(43) Date de publication de la demande: 13.11.1996
(73) Titulaire: Institut Français du Pétrole, 92502 Rueil-Malmaison (FR)
(72) Inventeur: Chodorge, Jean-Alain, 92160 Antony (FR); Commereuc, Dominique, 92190 Meudon (FR); Cosyns, Jean, 78580 Maule (FR); Duee, Didier, 95610 Eragny sur Oise (FR); Torck, Bernard, 92100 Boulogne sur Seine (FR)
(74) Mandataire: Hartmann, Günter

(56) Documents cités:
- EP-A- 0 605 822
- WO-A-94/08922
- US-A- 4 795 732
- US-A- 5 023 389
- US-A- 5 300 718
- S. Chaumette et al, Rev. Inst. Fr. Pet., 49(6), 1994, pp. 639-665

## Description

L'objet de la présente invention est un procédé de conversion d'au moins une coupe choisie dans le groupe formé par les coupes C₄ oléfiniques et les coupes C₅ oléfiniques en, d'une part, un éther (un alkyl-tertiobutyléther ou un alkyl-tertioamyléther) et d'autre part en propylène. Lorsque cette coupe est issue d'une opération de vapocraquage, un autre objet de la présente invention est l'optimisation de la sélectivité relative éthylène-propylène du vapocraquage grâce à la mise en oeuvre de ce procédé.

Le vapocraquage de charges constituées par des coupes paraffiniques légères fournit l'éthylène et le propylène nécessaires à la pétrochimie. Il fournit également un certain nombre d'autres produits plus lourds, et en particulier une coupe d'hydrocarbures en C₄ qui contient principalement du butadiène-1,3, de l'isobutène, des n-butènes et des butanes, accompagnés de traces d'hydrocarbures acétyléniques, et une coupe d'hydrocarbures en C₅ qui contient principalement des dioléfines en C₅, des méthylbutènes, des n-pentènes et des pentanes, accompagnés de traces d'hydrocarbures acétyléniques.

Le craquage catalytique, par exemple en lit fluide (FCC), de charges d'hydrocarbures lourds, fournit, à côté des fractions essence et gazole qui sont les produits principaux, des produits plus légers, parmi lesquels une coupe d'hydrocarbures en C₄ qui contient principalement de l'isobutane, de l'isobutène, des n-butènes et des butanes, accompagnés de faibles quantités de butadiène-1,3 et d'hydrocarbures acétyléniques, et une coupe d'hydrocarbures en C₅ qui contient principalement des pentanes, des méthylbutènes et des n-pentènes, accompagnés de faibles quantités de dioléfines en C₅ et d'hydrocarbures acétyléniques.

Jusqu'à récemment, seuls le butadiène-1,3 et l'isobutène trouvaient un usage dans l'industrie des polymères, en particulier dans l'industrie des pneumatiques pour le premier. L'augmentation de la longévité des pneumatiques et une relative stagnation de la demande font qu'il existe maintenant des surplus de butadiène qui ne sont pas, ou mal, utilisés. Au contraire, il y a un regain d'intérêt pour l'isobutène qui peut être mis en oeuvre dans la synthèse d'éthers utilisables comme additifs dans les carburants automobiles.

On connaît déjà des procédés pour produire un éther et du propylène à partit de coupes C₄.

Le brevet US-5 300 718 décrit un procédé pour produire un éther et du propylène par application des étapes successives suivantes :
a) éthérification,
b) extraction au solvant du butadiène,
c) séparation des impuretés oxygénées par adsorbant,
d) isomérisation du butène-1 en butène-2,
e) métathèse.

Le brevet EP-A- 605 822 décrit un procédé pour produire un éther à partir de composés C4 par application des étapes successives suivantes :
a') hydrogénation sélective,
b') éthérification,
c') séparation paraffineslotéfines (extraction au solvant + strippage) incluant une séparation des composés oxygénés,
d') isomérisation squeletaile pour produire de l'isobutène,
e') recyclage de l'isobutène vers l'étape b'.

La présente invention propose un procédé différent.

La présente invention propose un procédé de traitement d'une coupe d'hydrocarbures en C₄ contenant principalement de l'isobulène, des n-butènes, des butanes, et du butadiène-1,3 en quantité variable, qui inclut la transformation de l'isobutène en éthers (MTBE par exemple), et qui permet de transformer le butadiène-1,3 et les n-butènes en propylène utilisable par exemple pour la polymérisation. Elle propose également un procédé de traitement d'une coupe C₅ pour la transformer essentiellement en éthers (TAME par exemple) et en propylène. Il est également proposé un procédé de traitement combiné d'une coupe C₄ et d'une coupe C₅.

Les proportions relatives d'éthylène et de propylène produits dans une opération de vapocraquage peuvent être modulées dans une certaine mesure en changeant la nature de la charge et en modifiant les conditions opératoires (la sévérité) du craquage. Cependant, dans un mode de fonctionnement orienté vers une proportion plus grande de propylène, il se fait inévitablement des quantités plus importantes de coupe C₄, de coupe C₅ et de fractions plus lourdes d'essences de mauvaise qualité.

Un autre but de la présente invention est d'optimiser la sélectivité relative éthylène-propylène du vapocraquage grâce au traitement des coupes d'hydrocarbures en C₄ ou en C₅ qui produit entre autres du propylène, ce qui permet d'ajuster les proportions relatives d'éthylène et de propylène sans être obligé de changer la sévérité du craquage.

Le procédé objet de l'invention est plus précisément un procédé de conversion d'au moins une coupe oléfinique choisie dans le groupe formé par une coupe C₄ et une coupe C₅, en éther et en propylène, lesdites coupes contenant notamment des dioléfines, des alphaoléfines, des isooléfines et des impuretés acétylèniques, ledit procédé comportant les étapes suivantes se déroulant successivement:
1 ) hydrogénation sélective des dioléfines et des impuretés acétyléniques avec simultanément isomérisation des oléfines alpha en oléfines internes, par passage de ladite coupe en phase liquide sur un catalyseur comprenant au moins un métal choisi dans le groupe formé par le nickel, le palladium et le platine, déposé sur un support, à une température de 20-200 °C, une pression de 1-5 MPa, une vitesse spatiale de 0,5-10 h⁻¹, avec un rapport H₂ / dioléfines (molaire) de 0,5 à 5 et de préférence de 1 à 3, de façon à obtenir un effluent contenant majoritairement des oléfines internes et des isooléfines, et ne contenant pratiquement pas de dioléfines ni de composés acétyléniques,
2) étherification des isooléfines par réaction de l'effluent obtenu dans l'étape précédente avec un alcool, en présence d'un catalyseur acide, à une température de 30-130 °C, à une pression telle que la réaction se déroule en phase liquide, une séparation de l'éther et de l'alcool ayant lieu simultanément ou après l'étherification de façon à obtenir un effluent contenant majoritairement des oléfines internes accompagnées d'impuretés oxygénées,
3) séparation des impuretés oxygénées de l'effluent issu de l'étape 2), sur une masse de captation choisie dans le groupe formé par l'alumine, la silice, les silice-alumines, les zéolithes, à une température comprise entre 0 et 100°C, une pression de 0,3 à 5 MPa,
4) métathèse de l'effluent issu de l'étape précédente avec de l'éthylène, en présence d'un catalyseur comportant au moins un oxyde de rhénium déposé sur un support, à une température comprise entre 0 et 100 °C, et une pression au moins égale à la tension de vapeur du mélange réactionnel à la température dé réaction, de façon à obtenir un effluent contenant du propylène, la métathèse étant suivie d'une séparation du propylène.

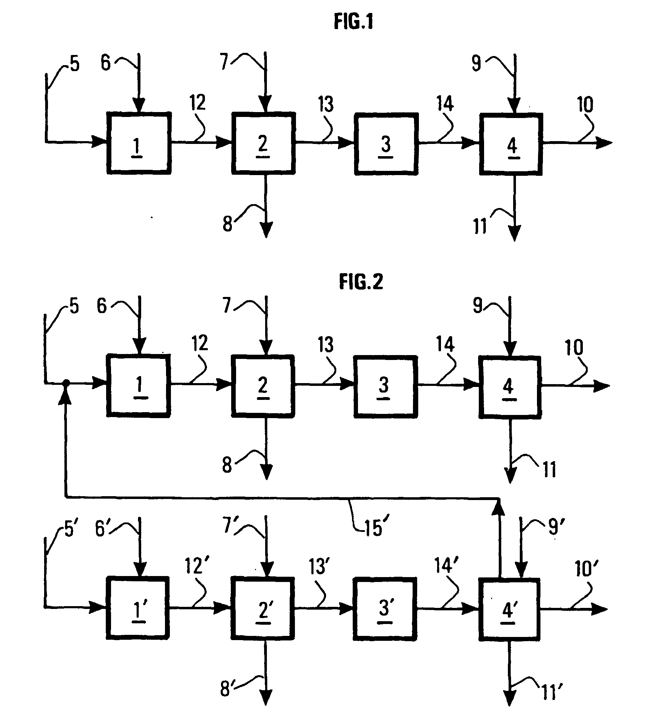

On décrira plus en détaille procédé selon l'invention schématisé sur la figure 1, à partir d'une coupe d'hydrocarbures en C₄ entrant par un conduit 5, qui contient principalement de l'isobutène, des n-butènes, des butanes, ainsi que du butadiène-1,3 en quantité variable. La coupe C₄ est soumise à une succession de traitements regroupés dans les étapes suivantes, pour produire un alkyl-tertiobutyléther et du propylène :
- hydroisomérisation sélective du butadiène-1,3 avec isomérisation du butène-1 en butène-2 et hydrogénation des hydrocarbures acétyléniques,
- étherification par un alcool produisant un alkyl-tertiobutyléther,
- séparation des composés oxygénés,
- métathèse de l'effluent riche en butène-2 en présence d'éthylène (éthènolyse) produisant du propylène.

La succession des traitements retenue dans le procédé selon l'invention présente de nombreux avantages. Les composés les plus réactifs de la coupe, à savoir les dioléfines (le butadiène-1,3 par exemple) en quantité variable, ainsi que les traces d'hydrocarbures acétyléniques sont transformés dès la première étape, et ne seront donc pas la cause de réactions parasites dans les étapes suivantes. Par ailleurs l'hydroisomérisation sélective des dioléfines (butadiène-1,3 par exemple) dans les coupes de vapocraquage permet d'augmenter considérablement la concentration en oléfine interne correspondante (butène-2 dans ce cas) dans la coupe, ce qui favorise d'autant l'étape de métathèse et un rendement élevé en propylène.

L'objet principal de la première étape est de transformer le butadiène-1,3 et tes n-butènes en butène-2. En effet, le butène-2 est la source du propylène qui est produit dans la dernière étape de métathèse en présence d'éthylène. Le butène-1 ne donne pas de produit nouveau avec l'éthylène, et il réagit avec le butène-2 pour donner du propylène, mais aussi des pentènes indésirables. Il est donc souhaitable de maximiser le rendement en butène-2, c'est-à-dire de se rapprocher autant que possible de la proportion autorisée par la thermodynamique. Le deuxième objet de cette étape est d'éliminer les traces d'hydrocarbures acétyléniques toujours présentes dans ces coupes et qui sont des poisons ou des polluants pour les étapes ultérieures.

Dans cette première étape (zone 1), on réalise donc simultanément les réactions suivantes, en présence d'hydrogène amené par un conduit 6 :
- hydroisomérisation sélective du butadiène-1,3 en un mélange de n-butènes à l'équilibre thermodynamique,
- isomérisation du butène-1 en butène-2, également à l'équilibre thermodynamique,
- hydrogénation des traces d'hydrocarbures acétyléniques.

Ces réactions peuvent être réalisées au moyen de divers catalyseurs spécifiques comprenant un ou plusieurs métaux, par exemple du groupe 10 de la classification périodique (Ni, Pd, Pt), déposés sur un support. On met en oeuvre de préférence un catalyseur qui comprend au moins un composé de palladium fixé sur un support minéral réfractaire, par exemple sur une alumine. La teneur en palladium sur le support peut être comprise entre 0,01 et 5 % en poids, de préférence entre 0,05 et 1 % en poids. Divers modes de prétraitement connus de l'homme de l'art peuvent éventuellement être appliqués à ces catalyseurs pour améliorer la sélectivité dans l'hydrogénation du butadiène-1,3 en butènes aux dépens de l'hydrogénation totale en butane qu'il faut éviter. Le catalyseur contient de préférence 0,05 à 10 % en poids de soufre. De façon avantageuse, on utilise un catalyseur constitué par du palladium déposé sur de l'alumine et qui contient du soufre.

La sulfuration du catalyseur peut être effectuée in situ (dans la zone de réaction) ou mieux ex situ. Dans ce dernier cas, on opère avantageusement selon le procédé décrit dans la brevet FR-93/09524, c'est-à-dire que le catatyseur a été traité, avant son chargement dans le réacteur d'hydrogénation, par au moins un composé soufré dilué dans un solvant, et que le catalyseur obtenu contenant 0,05 à 10 % de soufre (en poids) est chargé dans le réacteur et activé sous atmosphère neutre ou réductrice à une température comprise entre 20 et 300 °C, une pression comprise entre 0,1 et 5 MPa et une WH comprise entre 50 et 600 h⁻¹, et que la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion de butadiène-1,3 dans la coupe est importante, ce qui est le cas par exemple d'une coupe de vapocraquage lorsqu'on ne souhaite pas en extraire le butadiène-1,3 pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie théorique.

Les conditions opératoires sont choisies de telle façon que les réactifs et les produits soient en phase liquide. Il peut cependant être avantageux de choisir un mode de fonctionnement tel que les produits soient partiellement vaporisés à la sortie du réacteur, ce qui facilite le contrôle thermique de la réaction. La température peut varier de 20 à 200 °C, de préférence de 50 à 150 °C ou mieux de 60 à 150 °C. La pression peut être ajustée entre 0,1 et 5 MPa, de préférence entre 0,5 et 4 MPa et avantageusement entre 0,5 et 3 MPa, de telle façon que les réactifs, au moins en partie, soient en phase liquide. La vitesse spatiale peut être comprise entre 0,5 et 10 h⁻¹ et de préférence entre 1 et 6 h⁻¹, avec un rapport H₂ / dioléfines (molaire) de 0,5 à 5 et de préférence de 1 à 3.

Le ou les réacteurs d'hydroisomérisation peuvent avantageusement être suivis d'une colonne de stabilisation qui élimine les traces d'hydrogène en excès et le méthane éventuel.

L'objet de la deuxième étape (zone 2) est de transformer l'isobutène présent dans la coupe C₄ issue de l'étape précédente en un alkyl-tertiobutyléther par étherification avec un alcool entrant par un conduit 7, dont la chaîne hydrocarbonée peut comporter de 1 à 10 atomes de carborie. On utilise de préférence comme alcool le méthanol ou l'éthanol. Les éthers produits sont respectivement le méthyl-tertiobutyléther (MTBE) et l'éthyl-tertiobutyléther (ETBE), qui sortent par un conduit 8.

La transformation est réalisée au moyen d'un catalyseur acide, par exemple un catalyseur à base d'une résine échangeuse d'ions sous forme H⁺, comme une résine sulfonique -SO₃H. Ce catalyseur peut être mis en oeuvre par exemple en lit fixe de façon conventionnelle, ou en lit mobile. On préfère opérer avec un lit expansé de catalyseur, entretenu par une circulation à flux ascendant du milieu réactionnel à travers le réacteur et un échangeur de chaleur extérieur, Cette façon d'opérer permet de contrôler aisément le fonctionnement du catalyseur ainsi que l'élimination des calories produites dans la réaction, en évitant la formation de points chauds.

Un réacteur de finition peut avantageusement être placé à la suite du réacteur à lit expansé pour épuiser au maximum l'isobulène dans la coupe résiduelle et augmenter le rendement en éther, mais la majeure partie de la réaction se produit dans le réacteur à lit expansé. On peut également épuiser au maximum l'isobutène en mettant en oeuvre, en finition, une distillation réactive (colonne de distillation contenant du catalyseur) qui permet d'augmenter la conversion grâce à la séparation des produits au niveau du réacteur.

Les conditions opératoires sont choisies de telle façon que les réactifs et les produits soient en phase liquide. On fixe en général la température pour que la réaction se déroule à une vitesse suffisante, par exemple de 30 à 130 °C, de préférence de 40 à 100 °C, et la pression est ajustée en conséquence de telle façon que les réactifs soient en phase liquide.

La section réactionnelle est suivie d'une section de distillation où on sépare l'éther en fond de colonne et un distillat comprenant la coupe C₄ résiduelle et l'alcool en excès ainsi que des traces d'autres composés oxygénés volatils. L'alcool est séparé par un lavage à l'eau et le mélange eau-alcool est distillé pour recycler l'alcool.

La coupe C₄ issue de l'étape d'étherification contient en faible quantité un certain nombre de composés oxygénés et soufrés qu'il est indispensable de séparer dans une troisième étape (zone 3) du procédé car ce sont des poisons qui réduisent l'efficacité des catalyseurs de métathèse.

Pour réaliser cette séparation, on fait passer la coupe C₄ issue dé l'étape d'étherification sur une masse de captation qui présente une affinité plus grande pour les composés oxygénés que pour les hydrocarbures oléfiniques ou saturés, et qui peut être constituée par des oxydes réfractaires et/ou des aluminosilicates qui présentent un caractère acide, à savoir l'alumine, la silice, les silice-alumines, les zéolithes. Dans le cas où l'étape d'étherification met en jeu le méthanol, il peut être avantageux, pour diminuer le volume de la masse de captation, de la faire précéder par une colonne de distillation qui sépare le diméthyléther sous-produit dans l'étherificafion, et qui sèche en même temps la coupe C₄.

La masse de captation est mise en oeuvre par exemple en lit fixe, et dans des conditions de température et de pression telles que la charge et l'effluent soient en phase liquide. La température est comprise entre 0 et 100 °C, de préférence entre 10 et 50 °C. La pression est comprise entre 0,3 et 5 MPa, de préférence entre 0,4 et 1 MPa. La vitesse spatiale est ajustée pour obtenir un degré de captation maximum des composés oxygénés et soufrés.

Lorsque la masse de captation est saturée par les composés oxygénés et/ou soufrés, il est nécessaire de la régénérer, par exemple par entraînement par un courant gazeux chaud des composés qui ont été adsorbés. Le gaz utilisé peut être par exemple de l'azote, du méthane ou tout gaz inerte vis-à-vis de la masse de captation. La température de régénération peut être comprise entre 200 et 500 °C, de préférence entre 250 et 450 °C. Afin de ne pas interrompre la marche en continu du procédé, on peut disposer deux masses de captation en fonctionnement alterné.

La coupe obtenue à l'issue de la succession des étapes précédentes contient en majorité des butanes et du butène-2. Dans la dernière étape (zone 4) du procédé, ce butène-2 est mis en réaction avec de l'éthylène amené par un conduit 9, pour donner du propylène par métathèse (sortant par un conduit 10). Un conduit 11 évacue les sous-produits séparés.

La réaction de métathèse de l'éthylène avec le butène-2 peut être catalysée par des oxydes métalliques variés déposés sur des supports. On utilise de préférence un catalyseur comportant au moins un oxyde de rhénium déposé sur un support composé par un oxyde réfractaire contenant au moins de l'alumine, qui présente un caractère acide, comme par exemple l'alumine elle-même, les silice-alumines ou les zéolithes.

On peut citer à titre d'exemple préféré les catalyseurs comportant de l'heptoxyde de rhénium déposé sur une alumine gamma analogue à celle utilisée dans les catalyseurs de reforming, comme décrits dans le brevet US 4 795 734. La teneur en rhénium (exprimée en rhénium métallique) peut être comprise entre 0,01 et 20 %, de préférence entre 1 et 15 % en poids. Les catalyseurs sont soumis par exemple à une activation thermique finale à une température comprise entre 400 et 1000 °C pendant une durée de 10 minutes à 5 heures sous une atmosphère non-réductrice.

Les catalyseurs comportant de l'heptoxyde de rhénium déposé sur une alumine peuvent aussi être modifiés par l'adjonction d'un oxyde d'un autre métal. De tels catalyseurs modifiés comprennent par exemple du rhénium à l'état d'oxyde, de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale, tel que décrit dans le brevet FR 2 709 125.

La réaction de métathèse est effectuée de préférence en phase liquide, en l'absence d'oxygène, de composés oxygénés et d'humidité, et à une température comprise entre 0 et 200 °C, de préférence entre 20 et 150 °C, sous une pression au moins égale à la tension de vapeur du mélange réactionnel à la température de réaction.

Le catalyseur peut être mis en oeuvre en lit fixe. Cependant, comme il doit être fréquemment régénéré, il est alors nécessaire de disposer d'au moins deux réacteurs en parallèle, l'un étant en fonctionnement pendant que l'autre est en régénération. On utilise de préférence un système de lit catalytique mobile comme il est décrit dans le brevet français FR 2 608 595. Le catalyseur est soutiré à intervalles de temps réguliers en bas du réacteur et transféré vers un système de régénération en continu, d'où il est renvoyé en haut du réacteur

Compte-tenu des limitations imposées par la thermodynamique, l'éthylène non converti est fractionné dans une première colonne de distillation et recyclé au réacteur de métathèse. Une deuxième colonne de distillation sépare le propylène, et une troisième colonne sépare les hydrocarbures en C₄ non convertis qui peuvent être recyclés au réacteur de métathèse, et une fraction plus lourde en faible quantité.

La description du procédé schématisé dans la figure 1, et qui vient d'être faite pour la coupe C₄ est tout-à-fait transposable à la coupe C₆. Dans la première étape, les pentadiènes sont hydrogénés sélectivement en mono- oléfines et les oléfines alpha sont isomérisées en oléfines internes, c'est-à-dire en pentène-2 et méthyl-2 butène-2. L'étherification produit un alkyl-tertioamyléther, et en particulier le TAME (tertioamylméthyléther) si la réaction est effectuée avec du méthanol. La métathèse avec l'éthylène conduit à un mélange de propylène, isobutène et butène-1. Le propylène est séparé des autres produits, par exemple par distillation.

Le procédé peut aussi être mis en oeuvre simultanément sur la coupe C₄ et sur la coupe C₅, selon les descriptions faites précédemment. Il peut être alors avantageux de mélanger avec la coupe C₄ la fraction contenant en majorité du butène-1 et de l'isobutène obtenue par distillation de l'effluent de la métathèse sur la coupe C₅, comme indiqué dans la figure 2.

La figure 2 illustre un procédé selon l'invention dans lequel la coupe C₄ et la coupe C₅ sont traitées simultanément et séparément.

La coupe C₅ entrant par un conduit 5' est soumise à une étape 1' d'hydrogénation sélective (zone 1') avec de l'hydrogène entrant par un conduit 6', puis à une étape 2' d'étherification (zone 2') avec un alcool entrant par un conduit 7' et produisant un alkyl-tertioamyléther sortant par un conduit 8', suivie d'une étape 3' de séparation des impuretés oxygénées (zone 3') et ensuite d'une étape 4' de métathèse (zone 4') avec de l'éthylène entrant par un conduit g' et produisant du propylène sortant par un conduit 10', des sous-produits séparés (conduit 11') et un mélange butène-1 et isobutène évacués par un conduit 15'.

La coupe C₄ est soumise aux étapes 1, 2, 3, 4 déjà décrites à l'aide de la figure 1. Les étapes 1, 2, 3, 4, et 1', 2', 3', 4' sont réalisées selon les modalités du procédé décrites précédemment.

Il entre dans l'étape 1 (zone 1) d'hydrogénation sélective d'une part la coupe C₄ à traiter, et d'autre part le mélange butène-1 et isobutène issu de l'étape 4'. Ledit mélange est introduit avec la coupe C₄ dans la zone 1 par la canalisation 15' par exemple.

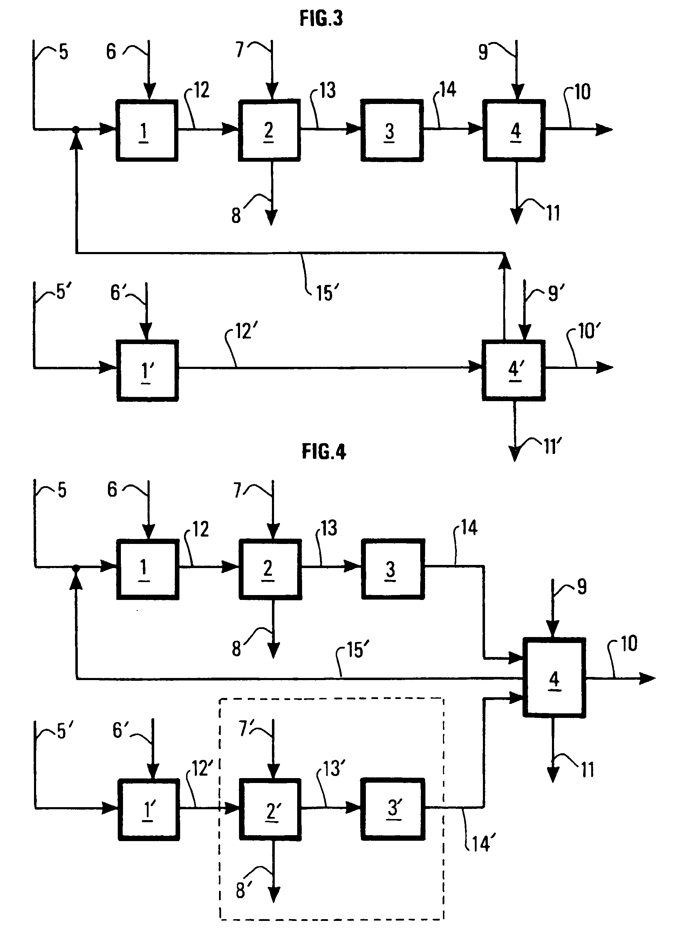

Dans ce cas, il peut être intéressant d'omettre les étapes d'étherification et de séparation des composés oxygénés dans la chaîne de traitement de la coupe C₅, de manière à accroître la quantité d'isobutène produit qui est renvoyé dans la chaîne de traitement de la coupe C₄. Ce mode de réalisation est illustré figure 3. On reconnaît les repères de la figure 2. La coupe C₄ et la coupe C₅ sont donc traitées simultanément et séparément La coupe C₅ n'est soumise qu'à une étape 1' (zone 1') d'hydrogénation sélective suivie d'une étape 4' (zone 4') de métathèse. Ces deux étapes ont lieu dans les conditions précédemment décrites. Il est donc obtenu du propylène, et un mélange d'isobutène et butène-1 qui est introduit avec la coupe C₄ dans l'étape 1. Il n'y a donc pas production d'alkyl-tertioamyléther.

D'une façon encore plus simplifiée, il peut être avantageux de mélanger les effluents des étapes 3 et 3' sur les deux chaînes C₄ et C₅ (ou l'effluent de l'étape 3 de la chaîne C₄ avec l'effluent de l'étape 1' de la chaîne C₅ si on omet sur cette chaîne l'étape d'étherification) de manière à ne conserver qu'une seule opération 4 de métathèse commune aux deux chaînes, comme indiqué dans la figure 4. Les pointillés symbolisent l'ensemble des étapes 2' et 3' qui peuvent ne pas être mises en oeuvre ainsi qu'illustré figure 3.

Lorsque le procédé est appliqué à une coupe C₄ ou C₅ de vapocraquage, il peut être avantageux d'intégrer l'unité de métathèse avec le craqueur, de manière à bénéficier du train de fractionnement de celui-ci.

Pour mettre en oeuvre le procédé décrit ci-dessus une installation est proposée (fig.1) qui comporte successivement:
- une zone 1 d'hydrogénation sélective avec isomérisation simultanée des oléfines alpha en oléfines internes, ladite zone comportant au moins un moyen 5 pour l'introduction de la coupe à convertir, au moins un moyen 12 pour la sortie de l'effluent et au moins un moyen 6 pour l'introduction de l'hydrogène, ladite zone comportant également au moins un lit de catalyseur qui comprend de préférence au moins un métal choisi dans le groupe formé par le nickel, le palladium et le platine, déposé sur un support,
- une zone 2 d'étherification et de séparation, comportant au moins un moyen 12 pour l'introduction de l'effluent issu de la zone 1, au moins un moyen 7 pour l'introduction de l'alcool, au moins un moyen 8 pour la sortie de l'alcool et/ou de l'éther, au moins un moyen 13 pour la sortie de l'effluent produit pratiquement sans alcool ni éther, la zone d'éthorification contenant au moins un catalyseur acide,
- une zone 3 de séparation des impuretés oxygénées, contenant au moins une masse de captation desdits composés, et comportant au moins un moyen 13 pour rintroduction de l'effluent issu de la zone 2, et au moins un moyen 14 pour la sortie de l'effluent produit, cette masse de captation étant choisie dans le groupe formé par l'alumine, la silice, les silice-alumines, les zéolites
- une zone 4 de métathèse contenant au moins un catalyseur de préférence à base d'oxyde de rhénium déposé sur un support, et comprenant au moins un moyen 14 d'introduction de l'effluent issu de la zone 3, au moins un moyen 9 d'introduction de l'éthylène, au moins un moyen 10 pour la sortie du propylène et au moins un moyen 11 pour la sortie de sous-produits.

Selon le mode de réalisation illustré figure 2, l'installation comporte :
- une zone 1 d'hydrogénation sélective avec isomérisation simultanée des oléfines alpha en oléfines internes, ladite zone comportant au moins un moyen 5 pour l'introduction de la coupe C₄ à convertir et au moins un moyen 15' pour l'introduction d'un mélange isobulène et butène-1, au moins un moyen 12 pour la sortie de l'effluent et au moins un moyen 6 pour l'introduction de l'hydrogène, ladite zone comportant également au moins un lit de catalyseur qui comprend de préférence au moins un métal choisi dans la groupe formé par la nickel, le palladium et le platine, déposé sur un support,
- une zone 2 d'étherification et de séparation, comportant au moins un moyen 12 pour l'introduction de l'effluent issu de la zone 1, au moins un moyen 7 pour l'introduction de l'alcool, au moins un moyen 8 pour la sortie de l'alcool et/ou de l'éther, au moins un moyen 13 pour la sortie de l'effluent produit pratiquement sans alcool ni éther, La zone d'étherification contenant au moins un catalyseur acide,
- une zone 3 de séparation des impuretés oxygénées, contenant au moins une masse de captation desdits composés, et comportant au moins un moyen 13 pour l'introduction de l'effluent issu de la zone 2, et au moins un moyen 14 pour la sortie de l'effluent produit, cette masse de captation étant choisie dans le groupe formé par l'alumine, la silice, les silice-alumines, les zéolites
- une zone 4 de métathèse contenant au moins un catalyseur de préférence à base d'oxyde de rhénium déposé sur un support, et comprenant au moins un moyen 14 d'introduction de l'effluent issu de la zone 3, au moins un moyen 9 d'introduction de l'éthylène, au moins un moyen 10 pour la sortie du propylène' et au moins un moyen 11 pour la sortie de sous-produits.
- une zone 1' d'hydrogénation sélective avec isomérisation simultanée des oléfines alpha en oléfines internes, ladite zone comportant au moins un moyen 5' pour l'introduction de la coupe C₅ à convertir, au moins un moyen 12' pour la sortie de l'effluent et au moins un moyen 6' pour l'introduction de l'hydrogène, ladite zone comportant également au moins un lit de catalyseur qui comprend de préférence au moins un métal choisi dans le groupe formé par le nickel, le palladium et le platine, déposé sur un support,
- une zone 2' d'étherification et de séparation, comportant au moins un moyen 12' pour l'introduction de l'effluent issu de la zone 1', au moins un moyen 7' pour l'introduction de l'alcool, au moins un moyen 8' pour la sortie de l'alcool et/ou de l'éther, au moins un moyen 13' pour la sortie de l'effluent produit pratiquement sans alcool ni éther, la zone d'étherification contenant au moins un catalyseur acide,
- une zone 3' de séparation des impuretés oxygénées, contenant au moins une masse de captation desdits composés, et comportant au moins un moyen 13' pour l'introduction de l'effluent issu de la zone 2', et au moins un moyen 14' pour la sortie de l'effluent produit,
- une zone 4' de métathèse contenant au moins un catalyseur de préférence à base d'oxyde de rhénium déposé sur un support, suivie d'une zone de séparation, et comprenant au moins un moyen 14' d'introduction de l'effluent issu de la zone 3', au moins un moyen 9' d'introduction de l'éthylène, au moins un moyen 10' pour la sortie du propylène, au moins un moyen 15' pour la sortie d'un mélange de butène-1 et d'isobutène et au moins un moyen 11' pour la sortie de sous-produits.

L'instaltation illustrée figure 3 comporte les zones 1, 2, 3, 4, 1', 4' décrites ci-dessus par la figure 2, et ne comporte pas les zones 2' et 3'.

Sur la figure 4, on a représenté une installation selon les figures 2 et 3 mais dans laquelle la zone 4 de métathèse est munie d'un moyen d'introduction 14 de l'effluent issu de la zone 3 et d'un moyen d'introduction 14' de l'effluent de la zone 3' (selon la figure 2) ou 12' de l'effluent de la zone 1' (selon la figure 3).

Dans une première réalisation, au moins un zone d'étherification et de séparation comprend une zone d'étherification suivie d'au moins une zone de séparation. Avantageusement, la zone d'étherification contient un lit expansé de catalyseur.

Dans une réalisation préférée, au moins un zone d'étherification et de séparation est une zone de distillation réactive contenant le catalyseur et dans laquelle ta séparation de l'éther et de l'alcool a lieu simultanément avec l'étherification.

On opère de préférence avec dans la zone de.métathèse un lit mobile de catalyseur.

De façon particulièrement intéressante, les coupes C₄ et C₅ proviennent d'une zone de vapocraquage en amont, le (les) moyen (s) d'introduction de la coupe à convertir dans la zone 1 (1') étant relié (s) à ladite zone de vapocraquage, et le moyen d'introduction de l'éthylène dans la zone 4 (4') étant relié à ladite zone de vapocraquage.

Les exemples suivants illustrent l'invention sans en limiter la portée.

### EXEMPLE 1

Une coupe C₄ en sortie de vapocraqueur présente la composition indiquée dans le tableau 1 (flux 1). Abréviations du tableau : MTBE = méthyl-tertiobutyléther, MAPD = mélhyl-acétylène + propadiène, BBV = butadiène-1,2 + butyne-1 + vinyl-acétylène, DME = diméthytéther.

Cette coupe C₄ est d'abord soumise à une hydroisomérisation. Elle est introduite en continu, avec le débit massique indiqué dans le tableau 1, et sous une pression de 2 MPa, dans un premier réacteur comprenant un lit fixe de 2,6 T d'un catalyseur constitué par du palladium sur alumine sulfuré au préalable. De l'hydrogène (mélangé à du méthane) est également injecté dans ce réacteur, comme indiqué dans le tableau 1 (flux 2). La température, qui est de 50°C à l'entrée, s'élève à 95 °C à la sortie. L'effluent est ensuite traité dans un réacteur definition chargé avec 2,5 T du même catalyseur, et fonctionnant pratiquement en isotherme à 70 °C. A la sortie (tableau 1, flux 3), la coupe est débarrassée des composés acétyléniques, et le butadiène-1,3 a été transformé essentiellement en butènes, qui sont en majorité des butènes-2, le butène-1 ayant été isomérisé. La coupe est alors traitée dans une colonne de stabilisation, où l'hydrogène résiduel et le méthane sont séparés. Après ce traitement, la coupe a la composition du flux 4 (tableau 1 ).

Dans la deuxième étape, la coupe est ethertfiée par du méthanol. Elle est mélangée avec du méthanol à l'entrée d'un groupé de deux réacteurs disposés en série et chargés avec 25 m³ d'un catalyseur constitué par une résine cationique sous forme acide sulfonique (Ambertyst 15 ou équivalente). Le mélange de la coupe et du méthanol (tableau 1, flux 5) est introduit en bas du premier réacteur qui fonctionne en lit expansé, avec une recirculation extérieure à travers un échangeur de chaleur, sous une pression de 1,9 MPa. La température est égale à 70 °C. L'effluent de ce premier étage entre dans le deuxième réacteur, qui fonctionne en lit fixe à 50 °C. A la sortie, le produit brut d'étherification (tableau 1, flux 6) est distillé pour séparer le MTBE et la coupe C₄ appauvrie en isobutène qui contient un peu de méthanol, et qui est lavée à l'eau pour en extraire le méthanol qui est recyclé. La coupe C₄ restante a la composition indiquée dans le tableau 1, flux 7.

Cette coupe contient des composés oxygénés en petites quantités, qui sont captés par passage à travers un lit de garde constitué par 20 T de tamis moléculaires de type 13X. L'adsorption se fait à une température voisine de 40 °C. La composition de l'effluent est donnée dans le tableau 1, flux 8.

Dans la dernière étape, la coupe résiduelle qui contient principalement des butènes-2 est mise en réaction avec de l'éthylène (composition globale : flux 9 du tableau 1 ) sur un catalyseur de métathèse, constitué par de l'oxyde de rhénium sur alumine gamma (8 % en poids en rhénium métal), préparé selon les enseignements du brevet US 4 795 734. La coupe C₄ est mélangée à l'entrée du réacteur de métathèse avec de l'éthylène d'appoint, ainsi qu'avec des flux de recyclage d'éthylène et de butènes. Ce réacteur fonctionne en lit mobile, comme décrit dans le brevet FR 2 608 595, à une température de 35 °C et sous une pression de 3,5 MPa, et il est couplé avec un régénérateur fonctionnant à 550 °C sous pression atmosphérique. Le catalyseur est soutiré à intervalles de temps réguliers en bas du réacteur et transféré vers le régénérateur, d'où il est renvoyé en haut du réacteur, les transferts se faisant à travers des sas tampons. En sortie de réacteur, l'éthylène non converti est fractionné dans une première colonne de distillation et recyclé. Une deuxième colonne de distillation sépare le propylène, et une troisième colonne sépare les hydrocarbures en C₄ non convertis qui sont également recyclés, et des pentènes en faible quantité. La composition de l'effluent de métathèse est indiquée dans le tableau 1, flux 10.

Le bilan global de la transformation s'établit donc comme suit. Pour 100 parties en poids (pp) de coupe C₄ sortie du vapocraqueur, on consomme 1,6 pp d'hydrogène, 14,9 pp de méthanol et 27,1 pp d'éthylène, et on produit 40,9 pp de MTBE et 81,6 pp de propylène. Au niveau du vapocraqueur d'où est issue la coupe C₄ traitée, ce bilan représente donc une faible consommation d'éthylène, qui permet une production supplémentaire de propylène importante, et ceci sans avoir à modifier les conditions de marche du craqueur.

### EXEMPLE 2

Une coupe C₅ en sortie de vapocraqueur présente la composition indiquée dans le tableau 2 (flux 1). Abréviations du tableau : TAME = méthyltertioamyléther, DME = diméthyléther.

Cette coupe C₅ est d'abord soumise à l'étape 1 d'hydrogénation et hydroisomérisation. Elle est introduite en continu, avec le débit massique indiqué dans le tableau 2, et sous une pression de 2 MPa, dans un premier réacteur comprenant un lit fixe de 1,2 T d'un catalyseur constitué par du palladium sur alumine sulfuré au préalable. De l'hydrogène (mélangé à du méthane) est également injecté dans ce réacteur, comme indiqué dans le tableau 2 (flux 2). La température, qui est de 50 °C à l'entrée, s'élève à 85 °C à la sortie. L'effluent est ensuite traité dans un réacteur de finition chargé avec 1,1 T du même catalyseur, et fonctionnant pratiquement en isotherme à 80 °C. A la sortie (tableau 2, flux 3), la coupe est débarrassée des traces de composés acétyléniques, et les dioléfines en C₅ ont été hydrogénées en méthyl-butènes et en pentènes, qui sont en majorité du méthyl-2 butène-2 et du pentène-2 en raison de l'isomérisation qui se fait en même temps. La coupe est alors traitée dans une colonne de stabilisation, où l'hydrogène résiduel et le méthane sont séparés. Après ce traitement, la coupe a la composition du flux 4 (tableau 2).

Dans la deuxième étape, la coupe est étherifiée par du méthanol. Elle est mélangée avec du méthanol à rentrée d'un groupe de deux réacteurs disposés en série et chargés avec 15 m³ d'un catalyseur constitué par une résine cationique sous forme acide sulfonique (Amberlyst 15 ou équivalente). Le mélange de la coupe et du méthanol (tableau 2, flux 5) est introduit en bas du premier réacteur qui fonctionne en lit expansé, avec une recirculation extérieure à travers un échangeur de chaleur, sous une pression de 1 MPa. La température est égale à 70 °C. L'effluent de ce premier étage entre dans le deuxième réacteur, qui fonctionne en lit fixe à 50 °C. A la sortie, le produit brut d'étherification (tableau 2, flux 6) est distillé pour séparer le TAME et la coupe C₅ appauvrie en méthyl-butènes qui contient encore un peu de méthanol. La coupe C₅ restante a la composition indiquée dans le tableau 2, flux 7.

Cette coupe contient des composés oxygénés en petites quantités, qui sont captés par passage à travers un lit de garde constitué par 10 T de tamis moléculaires de type 13X. L'adsorption se fait à une température voisine de 40 °C. La composition de remuent est donnée dans le tableau 2, flux 8.

Dans la dernière étape, la coupe résiduelle est mise en réaction avec de l'éthylène (composition globale: . flux 9 du tableau 2) sur un catalyseur de métathèse, constitué par de l'oxyde de rhénium sur alumine gamma (8 %'en poids en rhénium métal), préparé selon les enseignements du brevet US 4 795 734. Le réacteur de métathèse fonctionne en lit mobile, comme décrit dans le brevet FR 2 608 595, à une température de 35 °C et sous une pression de 3,5 MPa, et il est couplé avec un régénérateur fonctionnant à 550. °C sous pression atmosphérique. Le catalyseur est soutiré à intervalles de temps réguliers en bas du réacteur et transféré vers le régénérateur, d'où il ést renvoyé en haut du réacteur, les transferts se faisant à travers des sas tampons. En sortie de réacteur, l'éthylène non converti est fractionné dans une première colonne de distillation et recyclé. Une deuxième colonne de distillation sépare le propylène, et une troisième colonne sépare l'isobutène et le butène-1 en tête, et des oléfines plus lourdes en fond. La composition de l'affluent de métathèse est indiquée dans le tableau 2, flux 10.

Le bilan global de la transformation s'établit donc comme suit. Pour 100 parties en poids (pp) de coupe C₅ sortie du vapocraqueur, on consomme 1 pp d'hydrogène, 7,9 pp de méthanol et 8,5 pp d'éthylène, et on produit 25 pp de TAME, 8,3 pp de propylène, 4,4 pp d'isobutène et 6,7 pp de butène-1.

### EXEMPLE 3

Cet exemple décrit la mise en oeuvre à la fois d'une coupe C₄ et d'une coupe C₅ de vapocraquage pour produire du MTBE et du propylène.

La même coupe C₅ sortie de vapocraqueur que celle décrite dans l'exemple 2 est ici mise en oeuvre de façon à maximiser la quantité d'isobutène formé après métathèse, en omettant donc l'étape d'étherification, et par suite l'étape de séparation des oxygénés. Elle est ainsi soumise successivement à une étape d'hydroisomérisation, puis à une étape de métathèse en présence d'éthylène, dans les mêmes conditions et au moyen des mêmes catalyseurs que décrit dans l'exemple 2.

Le bilan de cette opération est donné dans le tableau 3. Le flux d'isobutène et de butène-1 obtenu après fractionnement de l'effluent brut de métathèse est alors mélangé avec la coupe C₄ mise en oeuvre dans l'exemple 1. Le mélange est soumis à la même succession d'étapes et dans les mêmes conditions que décrit dans l'exemple 1. Le bilan de l'opération est donné dans le tableau 4.

Le bilan global de la transformation s'établit donc comme suit. Pour 69 parties en poids (pp) de coupe C₄ et 31 parties en poids (pp) de coupe C₅ sortie du vapocraqueur, on consomme 1,5 pp d'hydrogène, 12,4 pp de méthanol et 23,8 pp d'éthylène, et on produit 34,2 pp de MTBE et 64,4 pp de propylène.

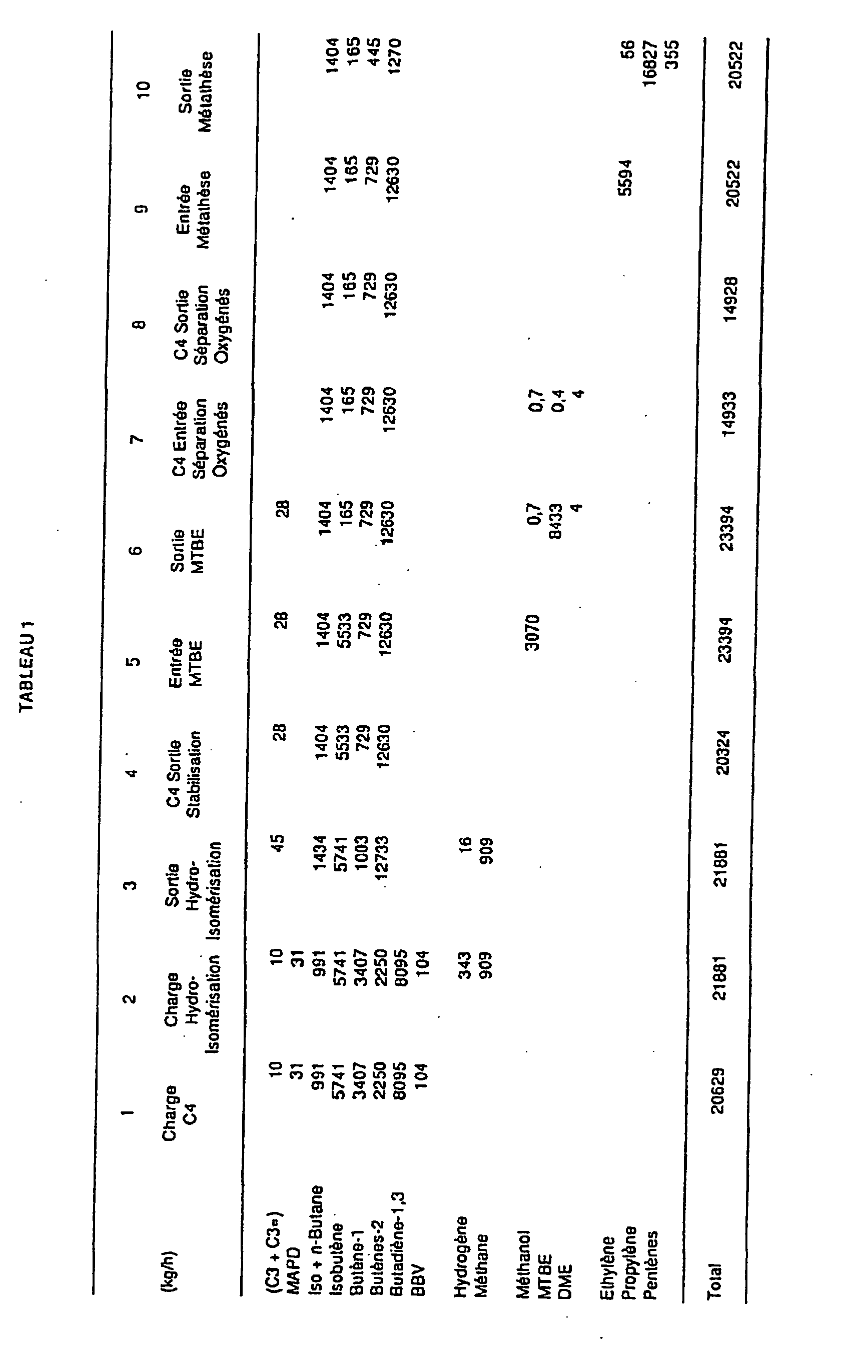

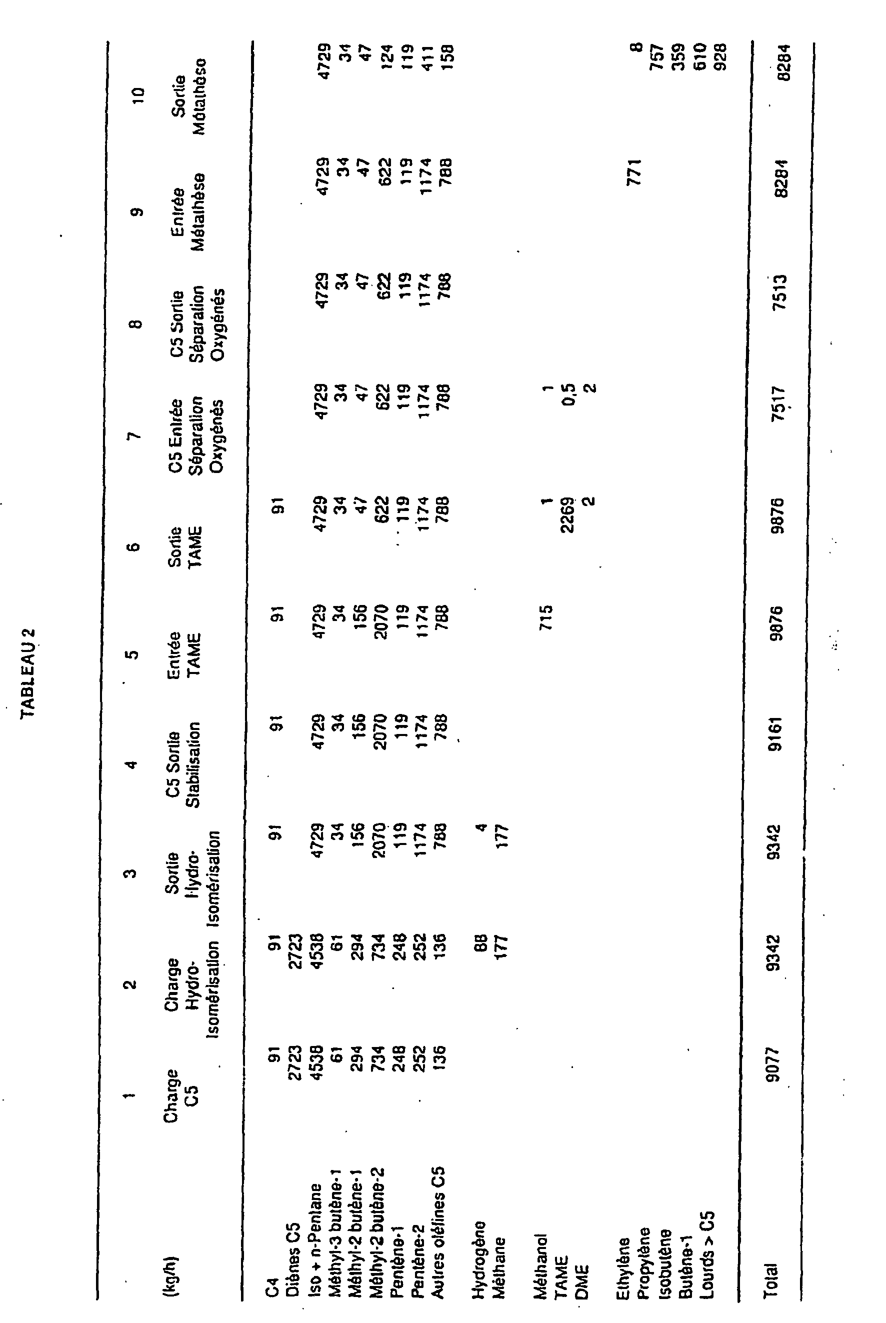

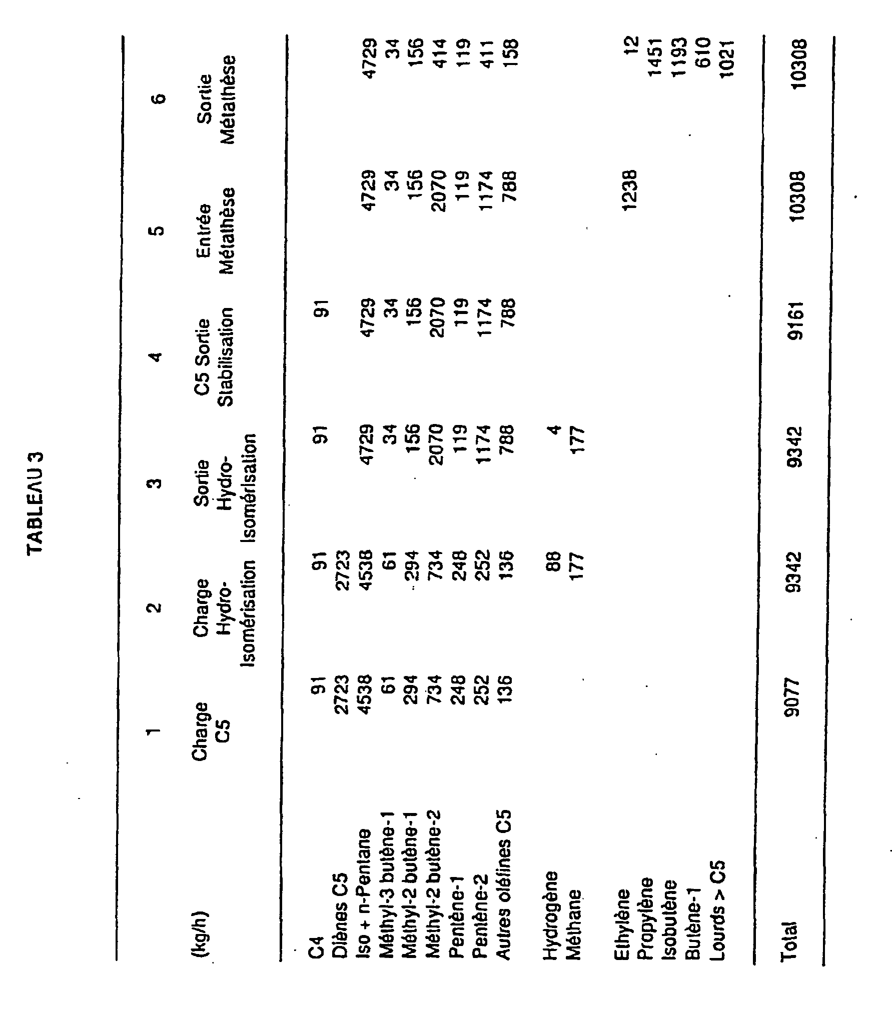

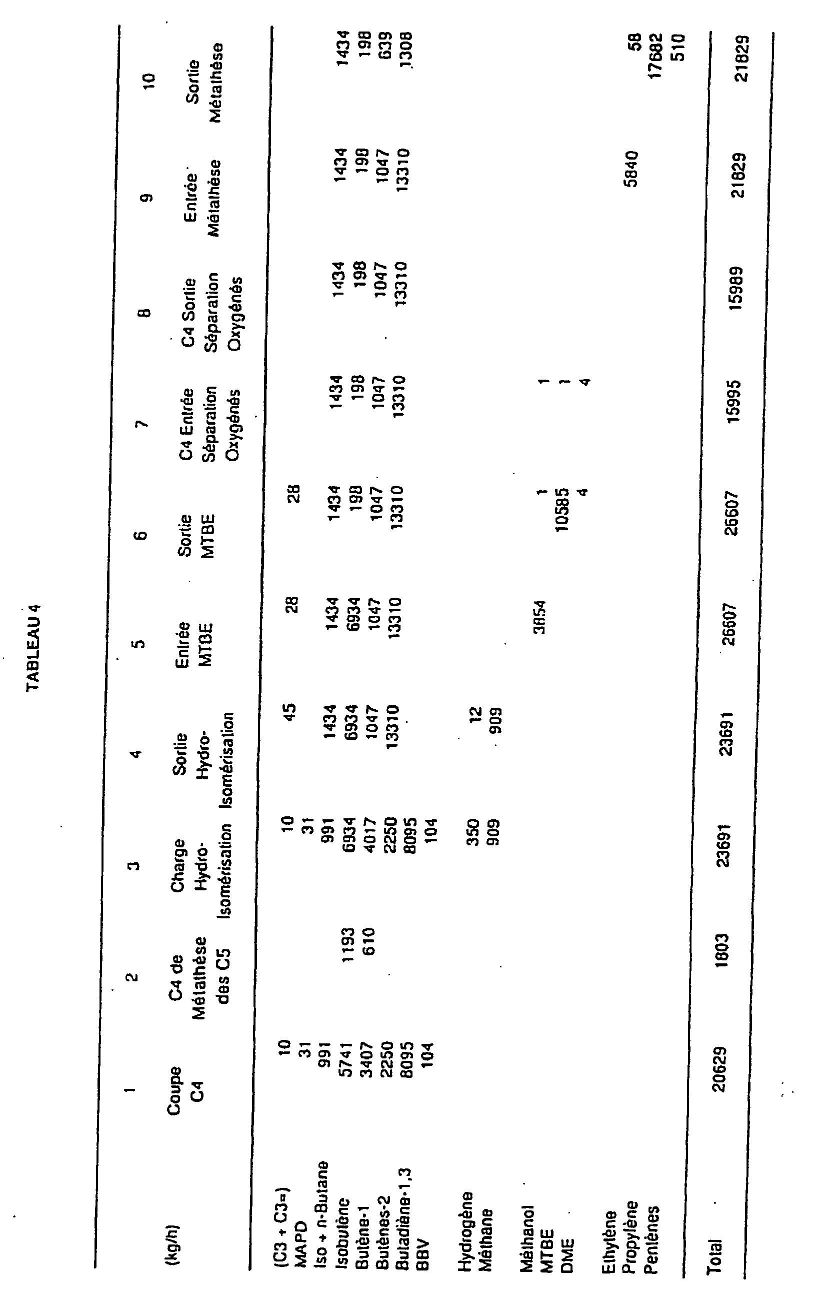

## Revendications

1. Procédé de conversion d'au moins une coupe oléfinique choisie dans le groupe formé par une coupe C₄ et une coupe C₅, en éther et en propylène, lesdites coupes contenant notamment des dioléfines, des alphaoléfines, des isooléfines et des impuretés acétyléniques, **caractérisé en ce que** ledit procédé comporte les étapes suivantes se déroulant successivement :
1) hydrogénation sélective des dioléfines et des impuretés acétyléniques avec simultanément isomérisation des oléfines alpha en oléfines internes, par passage de ladite coupe en phase liquide sur un catalyseur comprenant au moins un métal choisi dans le groupe formé par le nickel, le palladium et le platine, déposé sur un support, à une température de 20-200°C, une pression de 1-5 MPa, une vitesse spatiale de 0,5-10 h⁻¹, avec un rapport H₂/dioléfines (molaire) de 0, 5 à 5 et de préférence de 1 à 3, de façon à obtenir un effluent contenant majoritairement des oléfines internes et des isooléfines, et ne contenant pratiquement pas de dioléfines ni de composés acétyléniques,
2) étherification des isooléfines par réaction de l'effluent obtenu dans l'étape précédente avec un alcool, en présence d'un catalyseur acide, à une température de 30-130 °C, à une pression telle que la réaction se déroule en phase liquide, une séparation de l'éther et de l'alcool ayant lieu simultanément ou après l'étherification de façon à obtenir un effluent contenant majoritairement des oléfines internes accompagnées d'impuretés oxygénées,
3) séparation des impuretés oxygénées de l'effluent issu de l'étape 24 sur une masse de captation choisie dans le groupe formé par l'alumine, la silice, les silice-alumines, les zéolithes, à une température comprise entre 0 et 100 °C, une pression de 0,3 à 5 MPa.
4) métathèse de l'effluent issu de l'étape précédente avec de l'éthylène, en présence d'un catalyseur comportant au moins un oxyde de rhénium déposé sur un support, à une température comprise entre 0 et 100°C, et une pression au moins égale à la tension de vapeur du mélange réactionnel à la température de réaction, de façon à obtenir un effluent contenant du propylène, la métathèse étant suivie d'une séparation du propylène.

2. Procédé selon la revendication 1, **caractérisé en ce que** la coupe C₄ et la coupe C₅ sont traitées simultanément et séparément, que la coupe C₅ est soumise aux étapes 1' d'hydrogénation sélective, 2' d'étherirication, 3' de séparation des impuretés oxygénées et 4' de métathèse pour obtenir un alkyl-tertioamyléther, du propylène et un mélange d'isobutène et butène-1, et que ledit mélange d'isobutène et de butène-1 est introduit avec la coupe C₄ pour être soumis aux étapes 1 d'hydrogénation sélective, 2 d'étherification, 3 de séparation des impuretés oxygénées et 4 de métathèse pour produire un alkyl-tertiobutyléther et du propylène.

3. Procédé selon la revendication 1, **caractérisé en ce que** la coupe C₄ et la coupe C₅ sont traitées simultanément et séparément, que la coupe C₅ est soumise aux étapes 1' d'hydrogénation sélective et 4' de métathèse de l'effluent issu de l'étape 1' pour obtenir du propylène et un mélange d'isobutène et de butène-1, et que ledit mélange est introduit avec la coupe C₄ pour être soumis aux étapes 1, 2, 3 et 4 pour obtenir un alkyl-tertiobutyléther et du propylène.

4. Procédé selon la revendication 2, **caractérisé en ce que** l'étape 4 de métathèse traite simultanément les effluents issus des étapes 3 et 3'.

5. Procédé selon la revendication 3, **caractérisé en ce que** l'étape 4 de métathèse traite simultanément les effluents issus des étapes 3 et 1'.

6. Procédé selon l'une des revendications précédentes, **caractérisé en ce que** l'étape d'hydrogénation sélective se déroule en présence d'un catalyseur contenant 0.05 à 10 % en poids de soufre.

7. Procédé selon l'une des revendications précédentes, **caractérisé en ce que** le catalyseur a été traité, avant son chargement dans le réacteur d'hydrogénation, par au moins un composé soufré dilué dans un solvant, et que le catalyseur obtenu contenant 0,05 à 10 % de soufre (en poids) est chargé dans le réacteur et activé sous atmosphère neutre ou réductrice à une température comprise entre 20 et 300 °C, une pression comprise entre 0,1 et 5 . MPa et une WH comprise entre 50 et 600 h⁻¹, et que la charge est mise au contact dudit catalyseur activé.

8. Procédé selon l'une des revendications précédentes, **caractérisé en ce que** le catalyseur est constitué par du palladium déposé sur de l'alumine, et qui contient du soufre.

9. Procédé selon l'une des revendications 1 à 5, **caractérisé en ce que** l'étherification se déroule sur un catalyseur à base d'une résine échangeuse d'ions.

10. Procédé selon l'une des revendications 1 à 5 ou 9, **caractérisé en ce que** l'étherification se déroule avec un lit expansé de catalyseur.

11. Procédé selon l'une des revendications 1 à 5 ou 9, **caractérisé en ce que** l'étherification est mise en oeuvre dans une distillation réactive.

12. Procédé selon l'une des revendications 1 à 5, **caractérisé en ce que** la métathèse a lieu en présence d'un catalyseur contenant de l'oxyde de rhénium à raison de 0,01 à 20 % en poids exprimé en rhénium métallique, déposé sur un support contenant au moins 75 % en poids d'alumine et 0,01 à 30 % en poids d'au moins un oxyde d'un métal choisi dans le groupe formé par le niobium et le tantale.

13. Procédé selon l'une des revendications 1 à 5 ou 12, **caractérisé en ce que** la métathèse est effectuée avec un catalyseur en lit mobile.

14. Procédé selon l'une des revendications précédentes, **caractérisé en ce que** les coupes C₄ et C₅ sont issues d'une unité de vapocraquage.

15. Installation pour réaliser le procédé selon l'une des revendications 1 à 14 pour la conversion d'une coupe oléfinique choisie dans le groupe formé par une coupe C₄ et une coupe C₅, en éther et en propylène, **caractérisée en ce qu'**elle comporte successivement :
1) une zone 1 d'hydrogénation sélective avec isomérisation simultanée des oléfines alpha en oléfines internes, ladite zone comportant au moins un moyen 5 pour l'introduction de la coupe à convertir, au moins un moyen 12 pour la sortie de l'effluent, au moins un moyen 6 pour l'introduction de l'hydrogène, ladite zone comportant également au moins un lit de catalyseur,
2) une zone 2 d'étherification et de séparation, comportant au moins un moyen 12 pour l'introduction de l'ef-fluent issu de la zone 1, au moins un moyen 7 pour l'introduction de l'alcool, au moins un moyen 8 pour la sortie de l'alcool et/ou de l'éther, au moins un moyen 13 pour la sortie de l'effluent produit pratiquement sans alcool ni éther, la zone d'étherification contenant au moins un catalyseur acide,
3) une zone 3 de séparation des impuretés oxygénées, contenant au moins une masse de captation desdits composés, et comportant au moins un moyen 13 pour l'introduction de l'effluent issu de la zone 2, et au moins un moyen 14 pour la sortie de l'effluent produit, cette masse de captation étant choisie dans le groupe formé par l'alumine, la silice, les silice-alumines, les zéolithes
4) une zone 4 de métathèse contenant au moins un catalyseur suivie d'une zone de séparation, et comprenant' au moins un moyen 14 d'introduction de l'effluent issu de la zone 3, au moins un moyen 9 d'introduction de l'éthylène, au moins un moyen 10 pour la sortie du propylène et au moins un moyen 11 pour la sortie de sous-produits.

16. Installation selon la revendication 15, **caractérisée en ce qu'**elle comporte des zones successives 1 d'hydrogénation sélective, 2 d'étherification, 3 de séparation des impuretés oxygénées et 4 de métathèse pour traiter une coupe C₄ introduite par une canalisation 5 dans la zone 1, ladite installation comportant également des zones successives 1' d'hydrogénation sélective, 2' d'étherification, 3' de séparation des impuretés oxygénées et 4' de métathèse pour traiter une coupe C₅ introduite par une canalisation 5' dans la zone 1'. et la zone 4' étant munie d'une canalisation 15' de sortie d'un mélange butène-1 et isobutène reliée à la zone 1, de façon à introduire ledit mélange dans la zone 1.

17. Installation selon la revendication 15 **caractérisée en ce qu'**elle comporte des zones successives 1 d'hydrogéna-tion sélective, 2 d'étherification, 3 de séparation des impuretés oxygénées et 4 de métathèse pour traiter une coupe C₄ introduite par une canalisation 5 dans la zone 1, ladite installation comportant également des zones successives 1' d'hydrogénation sélective et 4' de métathèse pour traiter une coupe C₅ Introduite par une canalisation 5' dans la zone 1', et la zone 4' étant munie d'une canalisation 15' de sortie d'un mélange butène-1 et isobutène reliée à la zone 1, de façon à introduire ledit mélange dans la zone 1.

18. Installation selon la revendication 16, **caractérisée en ce qu'**elle comporte des zones successives 1, 2, 3, 4 pour traiter la coupe C₄ et des zones successives 1', 2', 3', 4 pour traiter la coupe C₅, la zone 4 étant munie d'un moyen d'introduction 14 de l'effluent issu de la zone 3 et d'un moyen d'introduction 14' de l'effluent issu de la zone 3'.

19. Installation selon la revendication 17, **caractérisée en ce qu'**elle comporte des zones successives 1, 2, 3, 4 pour traiter la coupe C₄ et des zones successives 1', 4 pour traiter la coupe C₅, la zone 4 étant munie d'un moyen d'introduction 14 de l'effluent issu de la zone 3 et d'un moyen d'introduction 12' de l'effluent issu de la zone 1'.

20. Installation selon l'une des revendications 15 à 19, **caractérisée en ce que** au moins une zone d'étherification et de séparation comprend une zone d'étherification suivie d'au moins une zone de séparation.

21. Installation selon rune des revendications précédentes, **caractérisée en ce que** la zone d'étherification contient un lit expansé de catalyseur.

22. Installation selon l'une des revendications 15 à 19, **caractérisée en ce que** au moins une zone d'étherification et de séparation est une zone de distillation réactive contenant le catalyseur et dans laquelle la séparation de réther et de l'alcool a lieu simultanément avec l'étherification.

23. Installation selon l'une des revendications précédentes, **caractérisée en ce que** la zone de métathèse contient un lit mobile de catalyseur.

24. Installation selon l'une des revendications précédentes, **caractérisée en ce que** le (les) moyen (s) d'introduction de la coupe à convertir est relié à une zone de vapocraquage produisant une coupe C₄ ou C₅, et **en ce que** le (les) moyen (s) d'introduction de l'éthylène dans la zone de métathèse est relié à la zone de vapocraquage.

## Patentansprüche

1. Verfahren zur Umwandlung wenigstens einer Olefinfraktion, ausgewählt aus einer durch eine C₄ und eine C₅-Fraktion gebildeten Gruppe in einen Ether und in Propylen, wobei diese Fraktionen insbesondere Diolefine, α-Olefine, Isoolefine und acetylenische Verunreinigungen enthalten, **dadurch gekennzeichnet, dass** dieses Verfahren die folgenden nacheinander ablaufenden Stufen umfasst:
1) selektive Hydrierung der Olefine und der acetylenischen Verunreinigungen unter gleichzeitiger Isomerisierung der α-Olefine zu internen Olefinen durch Überleiten dieser Fraktion in flüssiger Phase über einen Katalysator, der wenigstens ein Metall, abgeschieden auf einem Träger, enthält, das ausgewählt ist aus der durch Nickel, Palladium und Platin gebildeten Gruppe, bei einer Temperatur von 20-200°C, einem Druck von 1-5 MPa, einer Raumgeschwindigkeit von 0,5-10 h⁻¹ mit einem Verhältnis von H₂/Diolefine (Mol) von 0,5 bis 5, und bevorzugt von 1 bis 3, derart, dass ein Abstrom erhalten wird, der hauptsächlich interne Olefine und isoolefine und praktisch keine Diolefine noch acetylenische Verbindungen anhält,
2) Veretherung der isoolefine durch Reaktion des in der vorhergehenden Stufe erhaltenen Abstroms mit einem Alkohol in Anwesenheit eines sauren Katalysators bei einer Temperatur von 30-130° C und einem Druck, derart, dass die Reaktion in flüssiger Phase abläuft, wobei eine Abtrennung des Ethers und des Alkohols gleichzeitig mit der oder nach der Veretherung stattfindet, derart, dass ein Abstrom erhalten wird, der hauptsächlich interne Olefine, begleitet von sauerstoffhaltigen Verunreinigungen, enthält,
3) Abtrennung der sauerstoffhaltigen Verunreinigungen von dem aus der Stufe 2) stammenden Abstrom auf einer Einfarigmasse, ausgewählt aus einer Gruppe, die besteht aus Aluminiumoxid, Siliziumoxid, Siliziumoxid-Aluminiumoxiden und Zeolithen, bei einer Temperatur zwischen 0 und 100°C und einem Druck von 0,3 bis 5 MPa,
4) Metathesis des aus der vorhergehenden Stufe stammenden Abstroms mit Ethylen, in Anwesenheit eines Katalysators, der wenigstens ein Rheniumoxid, abgeschieden auf einem Träger, enthält, bei einer Temperatur, die zwischen 0 und 100°C liegt, und einem Druck, der wenigstens gleich dem Dampfdruck des Reaktionsgemisches bei. der Reaktionstemperatur ist, derart, dass ein Propylen enthaltender Abstrom erhalten wird, wobei sich an die Metathesis eine Abtrennung des Propylens anschließt.

2. Verfahren nach Anspruch 1, **dadurch gekennzeichnet, dass** die C₄-Fraktion und die C₅-Fraktion gleichzeitig und getrennt behandelt werden, dass die C₅-Fraktion in Stufen 1' der selektiven Hydrierung, 2' der Veretherung, 3' der Abtrennung der sauerstoffhaltigen Verunreinigungen und 4' der Metathesis ausgesetzt wird, um einen Alkyl-tert-amylether, Propylen und ein Gemisch aus isobuten und Buten-1 zu erhalten, und dass dieses Gemisch aus isobuten und Buten-1 mit der C₄-Fraktion eingeführt wird, um den Stufen 1 der selektiven Hydrierung, 2 der Veretherung, 3 der Abtrennung der sauerstoffhaltigen Verunreinigungen und 4 der Metathesis unterworfen zu werden, um Alkyl-tert-butylether und Propylen zu erzeugen.

3. Verfahren nach Anspruch 1, **dadurch gekennzeichnet, dass** die C₄-Fraktion und die C₅-Fraktion gleichzeitig und getrennt behandelt werden, dass die C₅-Fraktion den Stufen 1' der selektiven Hydrierung und 4' der Metathesis des aus der Stufe 1' stammenden Abstroms ausgesetzt wird, um Propylen und ein Gemisch aus Isobuten und Buten-1 zu erhalten, und dass dieses Gemisch mit der C₄-Fraktion eingeführt wird, um den Stufen 1, 2, 3 und 4 ausgesetzt zu werden und einen Alkyl-tert-butylether und Propylen zu erhalten.

4. Verfahren nach Anspruch 2, **dadurch gekennzeichnet, dass** die Stufe 4 der Metathesis gleichzeitig die aus den Stufen 3 und 3' stammenden Abströme behandelt.

5. Verfahren nach Anspruch 3, **dadurch gekennzeichnet, dass** die Metathesisstufe 4 gleichzeitig die aus den Stufen 3 und 1' stammenden Abströme behandelt.

6. Verfahren nach einem der vorhergehenden Ansprüche, **dadurch gekennzeichnet, dass** die Stufe der selektiven Hydrierung in Anwesenheit eines 0,05 bis 10 Gew% Schwefel enthaltenden Katalysators durchgeführt wird.

7. Verfahren nach einem der vorhergehenden Ansprüche, **dadurch gekennzeichnet, dass** der Katalysator vor seiner Aufgabe in den Hydrierungsreaktor durch wenigstens eine schwefelhaltige, in einem Lösungsmittel verdünnte Zusammensetzung behandelt wird, und dass der erhaltene 0,05 bis 10 (Gew-)% Schwefel enthaltende Katalysator in den Reaktor gegeben und in neutraler oder reduzierender Atmosphäre bei einer Temperatur zwischen 20 und 300°C, einem Druck zwischen 0,1 und 5 MPa und einer VVH zwischen 50 und 600 h⁻¹ aktiviert wird, und dass die Charge mit diesem aktivierten Katalysator kontaktiert wird.

8. Verfahren nach einem der vorhergehenden Ansprüche, **dadurch gekennzeichnet, dass** der Katalysator besteht aus auf Aluminiumoxid abgeschiedenem Palladium, welches Schwefel enthält.

9. Verfahren nach einem der Ansprüche 1 bis 5, **dadurch gekennzeichnet, dass** die Veretherung an einem Katalysator auf der Basis eines lonenaustauscherharzes abläuft.

10. Verfahren nach einem der Ansprüche 1 bis 5 oder 9, **dadurch gekennzeichnet, dass** die Veretherung mit einem expandierten oder geblähten Katalysalorbett abläuft.

11. Verfahren nach einem der Ansprüche 1 bis 5 oder 9, **dadurch gekennzeichnet, dass** die Veretherung in einer reaktiven Destillation verwirklicht wird.

12. Verfahren nach einem der Ansprüche 1 bis 5, **dadurch gekennzeichnet, dass** die Metathesis in Anwesenheit eines Katalysators stattfindet, der Rheniumoxid mit einem Anteil von 0,01 bis 20 Gew-%, ausgedrückt als metallisches Rhenium, enthält, welches auf einem Träger abgeschieden ist, der wenigstens 75 Gew % Aluminiumoxid und 0,01 bis 30 Gew.% wenigstens eines Oxids eines Metalls enthält, das aus der aus Niob und Tantal gebildeten Gruppe ausgewählt ist.

13. Verfahren nach einem der Ansprüche 1 bis 5 oder 12, **dadurch gekennzeichnet, dass** die Metathesis mit einem Katalysator im beweglichen Bett durchgeführt wird.

14. Verfahren nach einem der vorhergehenden Ansprüche, **dadurch gekennzeichnet, dass** die C₄- und C₅-Fraktionen aus einer Dampfcrack-Einheit stammen.

15. Anlage zur Durchführung des Verfahrens nach einem der Ansprüche 1 bis 14 zur Umwandlung einer Olefinfraktion, ausgewählt aus einer C₄- und einer C₅-Fraktion, in einen Ether und in Propylen, **dadurch gekennzeichnet, dass** sie aufeinanderfolgend umfasst:
1) eine Zone 1 der selektiven Hydrierung unter gleichzeitiger Isomerisierung der α-Olefine zu internen Olefinen, wobei diese Zone wenigstens ein Mittel 5 zum Einführen der umzuwandelnden Fraktion, wenigstens ein Mittel 12 für den Austritt des Abstroms, wenigstens ein Mittel 6 zum Einführen des Wasserstoffs umfasst, wobei diese Zone auch wenigstens ein Katalysatorbett umfasst,
2) eine Zone 2 zur Veretherung und Abtrennung, die enthält wenigstens ein Mittel 5 zum Einführen des aus der Zone 1 stammenden Abstroms, wenigstens ein Mittel 7 zum Einführen des Alkohols, wenigstens ein Mittel 8 für den Austritt des Alkohols und/oder des Ethers, wenigstens ein Mittel 13 für den Austritt des erzeugten Abstroms, der praktisch keinen Alkohol und Ether enthält, wobei die Veretherungszone wenigstens einen sauren Katalysator enthält,
3) eine Zone 3 zur Abtrennung der sauerstoffhaltigen Verunreinigungen, die wenigstens eine Einfangmasse für diese Verunreinigungen und wenigstens ein Mittel 13 zum Einführen des aus der Zone 2 stammenden Abstroms sowie wenigstens ein Mittel 14 für den Austritt des erzeugten Abstroms enthält, wobei die Einfangmasse ausgewählt wird aus der Gruppe Aluminiumoxid, Siliziumoxid, der Siliziumoxid-Aluminiumoxide und der Zeolithe,
4) eine wenigstens einen Katalysator enthaltende Metathesiszone 4, gefolgt von einer Abtrennzone, die wenigstens ein Mittel 14 zum Einführen das aus der Zone 3 stammenden Abstroms, wenigstens ein Mittel 9 zum Einführen des Ethylens, wenigstens ein Mittel 10 für den Austritt des Propylens und wenigstens ein Mittel 11 für den Austritt der Nebenprodukte umfasst.

16. Anlage nach Anspruch 15, **dadurch gekennzeichnet, dass** sie die aufeinanderfolgenden Zonen 1 der selektiven Hydrierung, 2 der Veretherung, 3 der Abtrennung der sauerstoffhaltigen Verunreinigungen und 4 der Metathesis zur Behandlung einer C₄-Fraktion umfasst, die über eine Leitung 5 in die Zone 1 eingeführt wurde, wobei die Anlage auch die aufeinanderfolgenden Zonen 1' der selektiven Hydrierung, 2' der Veretherung, 3' der Abtrennung der sauerstoffhaltigen Verunreinigungen und 4' der Metathesis zur Behandlung einer C₅-Fraktion umfasst, die über eine Leitung 5' in die Zone 1' eingeführt wurde, wobei die Zone 4' mit einer Austrittsleitung 15' für ein Gemisch aus Buten-1 und Isobuten, verbunden mit der Zone 1, derart ausgestattet ist, dass dieses Gemisch in die Zone 1 eingeführt wird.

17. Anlage nach Anspruch 15, **dadurch gekennzeichnet, dass** sie die aufeinanderfolgenden Zonen 1 der selektiven Hydrierung, 2 der Veretherung, 3 der Abtrennung der sauerstoffhaltigen Verunreinigungen und 4 der Metathesis zur Behandlung einer C₄-Fraktion umfasst, die über eine Leitung 5 in die Zone 1 eingeführt wurde" wobei die Anlage auch die aufeinanderfolgenden Zonen 1' der selektiven Hydrierung und 4' der Metathesis zur Behandlung einer C₅-Fraktion umfasst, die über eine Leitung 5' in die Zone 1' eingeführt wurde, wobei diese Zone 4' mit einer Leitung 15' für den Austritt eines Gemisches aus Buten-1 und Isobuten, verbunden mit der Zone 1, derart ausgestattet ist, dass dieses Gemisch in die Zone 1 eingeführt wird.

18. Verfahren nach Anspruch 16, **dadurch gekennzeichnet, dass** sie die aufeinanderfolgenden Zonen 1, 2, 3 und 4 zur Behandlung der C₄-Fraktion und die aufeinanderfolgenden Zonen 1', 2', 3', 4 zur Behandlung der C₅-Fraktion umfasst, wobei die Zone 4 ein Mittel 14 zum Einführen des aus der Zone 3 stammenden Abstroms und ein Mittel 14' zum Einführen des aus der Zone 3' stammenden Abstroms umfaßt.

19. Anlage nach Anspruch 17, **dadurch gekennzeichnet, dass** sie die aufeinanderfolgende Zonen 1, 2, 3, 4 zur Behandlung der C₄-Fraktion und die aufeinanderfolgende Zonen 1', 4 zur Behandlung der C₅-Fraktion umfasst, wobei die Zone 4 mit einem Mittel 14 zum Einführen des aus der Zone 3' stammenden Absiroms und einem Mittel 12' zur Einführung des aus der Zone 1' stammenden Abstroms ausgestattet ist.

20. Anlage nach einem der Ansprüche 15 bis 19, **dadurch gekennzeichnet, dass** wenigstens eine Zone der Veretherung und Abtrennung eine Veretherungszone, gefolgt von wenigstens einer Abtrennzone, umfasst.

21. Anlage nach einem der vorhergehenden Ansprüche, **dadurch gekennzeichnet, dass** die Veretherungszone ein expandiertes Katalysatorbett enthält.

22. Anlage nach einem der Ansprüche 15 bis 19, **dadurch gekennzeichnet, dass** wenigstens die. Veretherungs- und Abtrennzone eine Zone der reaktiven Destillation ist, die den Katalysator enthält, in der die Abtrennung von Ether und Alkohol gleichzeitig mit der Veretherung stattfindet.

23. Anlage nach einem der vorhergehenden Ansprüche, **dadurch gekennzeichnet, dass** die Metathesiszone ein bewegliches Katalysatorbett enthält.

24. Anlage nach einem der vorhergehenden Ansprüche, **dadurch gekennzeichnet, dass** das (die) Mittel zur Einführung der umzuwandelnden Fraktion mit einer Dampfcrackzone verbunden ist, die eine C₄- oder C₅-Fraktion erzeugt, und dass das (die) Mittel zur Einführung des Ethylens in die Metathesiszone mit der Dampfcrackzone verbunden ist (sind).

## Claims

1. A process for the conversion of at least one olefinic cut, selected from the group formed by a C₄ cut and a C₅ cut, to an ether and to propylene, said cuts containing mainly diolefins, alpha olefins, isoolefins and acetylenic impurities, said process comprising the following steps which are carried out successively:
1) selective hydrogenation of the diolefins and acetylenic impurities with simultaneous isomerisation of the alpha olefins to internal olefins by passing said cut in the liquid phase over a catalyst comprising at least one metal selected from the group formed by nickel, palladium and platinum, deposited on a support, at a temperature of 20-200 °C, a pressure of 1-5 MPa, and a space velocity of 0.5-10 h⁻¹, with a H₂/diolefin (molar) ratio of 0.5 to 5, preferably 1 to 3, to obtain an effluent containing mainly internal olefins and isoolefins, and containing practically no diolefins or acetylenic compounds;
2) etherification of the isoolefins by reacting the effluent from the preceding step with an alcohol, in the presence of an acid catalyst, at a temperature of 30-130°C, at a pressure such that the reaction is carried out in the liquid phase, the ether and alcohol being separated simultaneously with or after etherification to obtain an effluent containing mainly internal olefins accompanied by oxygen-containing impurities;
3) separation of the oxygen-containing impurities from the effluent from step (2) on a captation mass selected from the group consisting of alumina, silica, silica-aluminas and zeolites, at a temperature in the range of 0°C to 100°C, and at a pressure of 0.3 to 5 MPa;
4) metathesis of the effluent from the preceding step with ethylene, in the presence of a catalyst comprising at least one rhenium oxide deposited on a support, at a temperature in the range of 0°C to 100°C, and at a pressure which is at least equal to the vapour tension of the reaction mixture at the reaction temperature, to obtain an effluent containing propylene, the metathesis being followed by separation of the propylene.

2. The process according to claim 1, **characterized in that** the C₄ cut and the C₅ cut are treated simultaneously and separately, wherein the C₅ cut undergoes a selective hydrogenation step 1', an etherification step 2', a step 3' for separating oxygen-containing impurities and a metathesis step 4' to obtain an alkyl-tertioamylether, propylene and a mixture of isobutene and 1-butene, and wherein said mixture of isobutene and 1-butene is introduced with the C₄ cut to undergo selective hydrogenation step 1, etherification step 2, step 3 for separating oxygen-containing impurities and metathesis step 4 to produce an alkyl-tert-iobutylether and propylene.

3. The process according to claim 1, **characterized in that** the C₄ cut and the C₅ cut are treated simultaneously and separately, wherein the C₅ cut undergoes a selective hydrogenation step 1' and a metathesis step 4' for the effluent from step 1' to obtain propylene and a mixture of isobutene and 1-butene, and wherein said mixture is introduced with the C₄ cut and undergoes steps 1, 2, 3 and 4 to obtain an alkyltertiobutylether and propylene.

4. The process according to claim 2, **characterized in that** metathesis step 4 simultaneously treats the effluents from steps 3 and 3'.

5. The process according to claim 3, **characterized in that** metathesis step 4 simultaneously treats the effluents from steps 3 and 1'.

6. The process according to any of the preceding claims, **characterized in that** the selective hydrogenation step is carried out in the presence of a catalyst containing 0.05% to 10% by weight of sulphur.

7. The process according to any of the preceding claims, **characterized in that** the catalyst has been treated, before charging it into the hydrogenation reactor, with at least one sulphur-containing compound diluted in a solvent, and **in that** the catalyst obtained containing 0.05% to 10% (by weight) of sulphur is charged into the reactor and activated in a neutral or reducing atmosphere at a temperature in the range of 20°C to 300°C, at a pressure in the range of 0.1 to 5 MPa and a GHSV in the range of 50 to 600 h⁻¹, and **in that** the feed is brought into contact with said activated catalyst.

8. The process according to any of the preceding claims, **characterized in that** the catalyst is constituted by palladium deposited on alumina and containing sulphur.

9. The process according to any of claims 1 to 5, **characterized in that** etherification is carried out using a catalyst based on an ion exchange resin.

10. The process according to any of claims 1 to 5 or 9, **characterized in that** etherification is carried out using an expanded catalyst bed.

11. The process according to any of claims 1 to 5 or 9, **characterized in that** etherification is carried out in a reactive distillation step.

12. The process according to any of claims 1 to 5, **characterized in that** metathesis takes place in the presence of a catalyst containing a rhenium oxide in a proportion of 0.01 % to 20% by weight, expressed as rhenium metal, deposited on a support containing at least 75% by weight of alumina and 0.01 % to.30% by weight of at least one oxide of a metal selected from the group formed by niobium and tantalum.

13. The process according to any of claims 1 to 5 or 12, **characterized in that** metathesis is carried out with a moving catalyst bed.

14. The process according to any of the preceding claims, **characterized in that** the C₄ and C₅ cuts originate from a steam cracking unit.

15. A plant for realizing the process according to any of claims 1 to 14 for converting an olefinic cut, selected from the group formed by a C₄ cut and a C₅ cut, to an ether and to propylene, **characterized in that** it comprises successively:
1) a zone 1 for selective hydrogenation with simultaneous isomerisation of alpha olefins to internal olefins, said zone comprising at least one means 5 for introducing a cut to be converted, at least one means 12 for discharging an effluent and at least one means 6 for introducing hydrogen, said zone also comprising at least one catalyst bed;
2) an etherification and separation zone 2 comprising at least one means 12 for introducing an effluent from zone 1, at least one means 7 for introducing an alcohol, at least one means 8 for discharging alcohol and/or an ether, and at least one means 13 for discharging an effluent which is practically free of alcohol and ether, the etherification zone containing at least one acid catalyst;
3) a zone 3 for separating oxygen-containing impurities, containing at least one mass for capturing said compounds, and comprising at least one means 13 for introducing an effluent from zone 2, and at least one means 14 for discharging the effluent produced, said captation mass being selected from the group consisting of alumina, silica, silica-aluminas und zeolites;
4) a metathesis zone 4 containing at least one catalyst, followed by a separation zone, and comprising at least one means 14 for introducing an effluent from zone 3, at least one means 9 for introducing ethylene, at least one means 10 for discharging propylene and at least one means 11 for discharging by-products.

16. The plant according to claim 15, **characterized in that** it successively comprises zones for selective hydrogenation 1, etherification 2, separating oxygen-containing impurities 3 and metathesis 4 for the treatment of a C₄ cut introduced via a line 5 into zone 1, said plant also successively comprising zones for selective hydrogenation 1', etherification 2', separating oxygen-containing impurities 3' and metathesis 4' to treat a C₅ cut introduced into zone 1' via a line 5', zone 4' being provided with a line 15' for discharging a mixture of 1-butene and isobutene which is connected to zone 1 to introduce said mixture into zone 1.

17. The plant according to claim 15, **characterized in that** it comprises successive zones for selective hydrogenation 1, etherification 2, separating oxygen-containing impurities 3 and metathesis 4 to treat a C₄ cut introduced into zone 1 via a line 5, said plant also comprising successive zones for selective hydrogenation 1' and metathesis 4' to treat a C₅ cut introduced via a line 15' for discharging a mixture of 1-butene and isobutene collected to zone 1, to introduce said mixture into zone 1.

18. The plant according to claim 16, **characterized in that** it comprises successive zones 1, 2, 3 and 4 to treat a C₄ cut and successive zones 1', 2', 3' and 4 to treat the C₅ cut, zone 4 being provided with means 14 for introducing an effluent from zone 3 and means 14' for introducing an effluent from zone 3'.

19. The plant according to claim 17, **characterized in that** it comprises successive zones 1, 2, 3 and 4 to treat the C₄ cut and successive zones 1' and 4 to treat the C₅ cut, zone 4 being provided with means 14 for introducing an effluent from zone 3 and means 12' for introducing an effluent from zone 1'.

20. The plant according to any of claims 15 to 19, **characterized in that** at least one etherification and separation zone comprises an etherification zone followed by at least one separation zone.

21. The plant according to any of the preceding claims, **characterized in that** the etherification zone contains an expanded catalyst bed.

22. The plant according to any of claims 15 to 19, **characterized in that** at least one etherification and separation zone is a reactive distillation zone containing the catalyst and in which separation of the ether and the alcohol takes place simultaneously with etherification.

23. The plant according to any of the preceding claims, **characterized in that** the metathesis zone contains a mobile catalyst bed.

24. The plant according to any of the preceding claims, **characterized in that** the introduction means for the cut to be converted is connected to a steam cracking zone producing a C₄ or a C₅ cut, and **in that** the means for introducing the ethylene into the metathesis zone is connected to the steam cracking zone.
